Europäisches Patentamt
European Patent Office
Office européen des brevets

(19)

(11) Publication number: **0 516 186 A2**

(12) **EUROPEAN PATENT APPLICATION**

(21) Application number: **92112530.8**

(22) Date of filing: **06.04.88**

(51) Int. Cl.[5]: **C07D 239/74**, C07D 239/54, C07D 409/04, C07H 19/06, A61K 31/70

This application was filed on 22 - 07 - 1992 as a divisional application to the application mentioned under INID code 60.

(30) Priority: **16.04.87 SE 8701605**

(43) Date of publication of application: **02.12.92 Bulletin 92/49**

(60) Publication number of the earlier application in accordance with Art.76 EPC: **0 309 560**

(84) Designated Contracting States: **AT BE CH DE FR GB IT LI LU NL SE**

(71) Applicant: **Medivir Aktiebolag**
**Lunastigen 7**
**S-141 44 Huddinge(SE)**

(72) Inventor: **Datema, Roelf**
**Sandoz Forschungsinst., Brunnerstrasse 59**
**A-1235 Wien(AT)**
Inventor: **Lindborg, Björn G.**
**Helgarögränd 14**
**S-125 42 Älvsjö(SE)**
Inventor: **Kovacs, Zsuzanna, M I**
**Via Nazario Sauro 118**
**I-170 27 Peitra Ligure(IT)**
Inventor: **Stening, Göran Bertil**
**Varbovägen 10**
**S-151 50 Södertälje(SE)**
Inventor: **Johansson, Karl Nils G.**
**Bäverstigen 19**
**S-150 23 Enhörna(SE)**
Inventor: **Öberg, Bo Fredrik**
**Askvägen 27**
**S-752 52 Uppsala(SE)**

(74) Representative: **Larfeldt, Helene (SE) et al**
**Bergenstrahle & Lindvall AB Box 17704**
**S-118 93 Stockholm(SE)**

(54) **Nucleosides and nucleoside analogues, pharmaceutical composition and processes for the preparation of the compounds.**

(57) A compound of a formula (I)

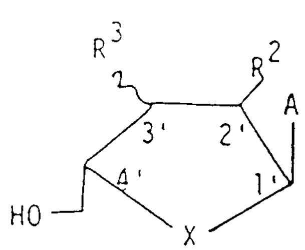

or

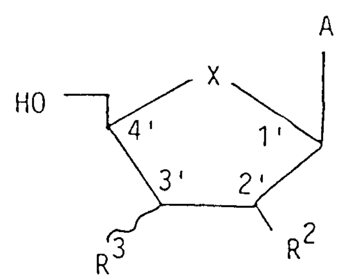

I

α-anomer

β-anomer

wherein the radicals A, X, $R^2$ and $R^3$ are defined as follows

EP 0 516 186 A2

**A:** (a) 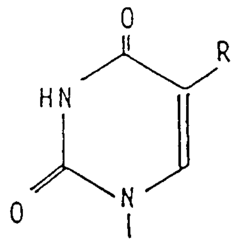 or (b) 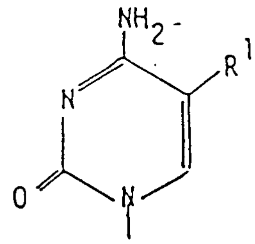

wherein $R^1$ is H, alkyl containing 1-3 carbon atoms, including cyclopropyl; $-CH=CH_2$; $-CH=CH-CH_3$; $-CH_2-CH=CH_2$;

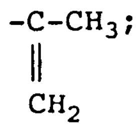

C≡CH

X:

(a) S

(b) $CH_2$

$R^2$: H; or $R^2$ constitutes together with $R^3$ a carbon - carbon bond

$R^3$: H; F; Cl; Br; I; $N_3$; CN; C≡CH; OH; $OCH_3$; $CH_2OH$; whereby when $R^3$ in formula I is F; Cl; Br; I; $N_3$; CN; C≡CH; OH; $OCH_3$ or $CH_2OH$ it may have either cis-configuration or trans-configuration relative to the hydroxymethyl function at position 4', or $R^3$ constitutes together with $R^2$ a carbon - carbon bond, and therapeutically acceptable salts thereof for use in therapy, in particular for the treatment of HIV infections.

Field of the invention

The present invention relates to the use of chemical compounds and physiologically acceptable salts thereof for the therapheutic and prophylactic control and treatment of the Acquired Immuno Deficiency Syndrome (AIDS), infections by Human Immunodeficiency Virus, hepatitis B virus infections and retrovirus infections and method for such control and treatment in animal and man.

Background of the invention

In the late seventies a new disease was reported, which subsequently was referred to as Acquired Immuno Deficiency Syndrome (AIDS). It is now generally accepted that a retrovirus referred to as HIV (Human Immuno-deficiency Virus), formerly known as Human T-cell Lymphotropic Virus (HTLV-III) or Lymphadenopathy Associated Virus (LAV) plays an essential role in the etiology of AIDS.

AIDS is characterized by a profound immunodeficiency due to low numbers of a subset of lymphocyte-T-helper cells, which are one target for HIV infection. The profound immunodeficiency in AIDS patients makes these patients highly susceptible to a variety of opportunistic infections of bacterial, fungal, protozoal or viral etiology. The etiological agents among viral opportunistic infections are often found in the herpes virus group, i .e., Herpes simplex virus (HSV), Varicella Zoster virus (VZV), Epstein-Barr virus (EBV) and, especially, cytomegalovirus (CMV). Other retroviruses affecting humans are HTLV-I and II and examples of retroviruses affecting animals are feline leukemia virus and equine infectios anaemia virus.

Hepatitis B virus infections cause severe disease such as acute hepatitis, chronic hepatitis, fulminant hepatitis in a considerable number of persons. It is estimated that there are 200 million patients with chronic hepatitis B infection in the world. A considerable number of the chronic cases progress to liver cirrosis and liver tumours. In some cases the hepatitis infections also take a rapid and severe course as in fulminant B hepatitis with about 90 % mortality. At present there is no known effective treatment against hepatitis B infections.

General outline of the invention

A great number of nucleoside analogues exhibit several antimetabolic activities. They do so by substituting for or competing with the naturally occuring nucleosides. Recently some nucleoside analogues have been described, which inhibit in cell culture the multiplication of human immunodeficiency virus (HIV, also called HTLV-III, LAV), the causative agent of AIDS and AIDS-related complex (ARC). Such compounds are for example azidothymidine, dideoxycytidine and dideoxyadenosine. These and other described HIV-antimetabolic nucleoside analogues have the same geometric relationship between the nucleoside base and the glycosidic part as the naturally occuring nucleosides, i.e. they are $\beta$-anomers.

We have now, surprisingly, found that some nucleosides and nucleoside analogues with the opposite geometric configuration, $\alpha$-anomers, are potent inhibitors of HIV multiplication but not of cell-division. Anti-HIV activities are displayed by such geometric isomers which have been modified either in the nucleoside base part, the glycoside part or in both parts. The structures of these compounds are disclosed in this invention.

Prior Art

The following compounds of the formula I below are known:

1. Compounds of the formula

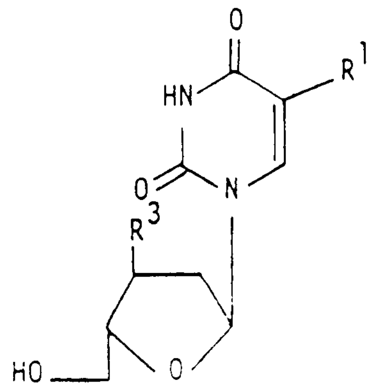

wherein $R^3$ is OH and $R^1$ is as follows:

| | |
|---|---|
| $R^1$ is H and $CH_3$: | T. Nishimura, B. Shinizu, I. Iwai<br>Chem. Pharm. Bull. (Tokyo) 12 (1964), 1471 |
| $R^1$ is $C_2H_5$: | M. Swierkowski, D. Shugar<br>J. Med. Chem. 12 (1969), 533 |
| $R^1$ is n-$C_3H_7$: | A. Szaboles, J. Sagi, L. Ötvös<br>J. Carbohydrates, Nucleosides, Nucleotides 2 (1975), 197 - 211 |
| $R^1$ is i-$C_3H_7$: | M. Draminski, A. Zgit-Wroblewska<br>Polish J. Chemistry 54 (1980), 1085 |
| $R^1$ is C≡CH: | P.J. Barr, A.S. Jones, P. Serafinowski, R. Walker<br>J. Chem. Soc. Perkin I (1978), 1263 - 1267 |

and wherein $R^3$ is $N_3$ and $R^1$ is $CH_3$: H. Imezawa, F. Eckstein, J. Org. Chem. 43 (1978), 3044-3048.

2. The compound of the formula

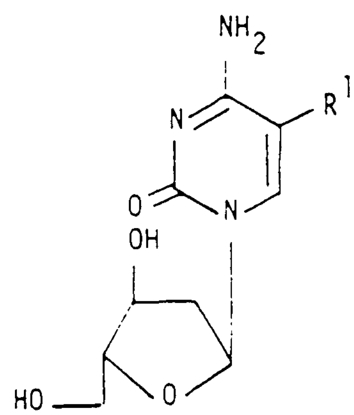

$R^1$ is C≡CH is described by P.J. Barr, A.S. Jones, P. Serafinowski, R. Walker, J. Chem. Soc. Perkin I (1978), 1263 - 1267 $R^1$ is H is described by J.J. Fox, N.C. Yung, I. Wempen and M. Hoffer, J. Am. Chem. Soc., Vol. 83 (1961), 4066-4070.

Both groups 1. and 2. concern only compounds having the 3' group and the 4'hydroxymethyl group in a trans-configuration.

## Disclosure of the invention

It has been found according to the present invention that the compounds of the formula

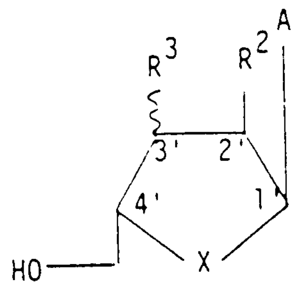

I

wherein the radicals A, X, $R^1$, $R^2$ and $R^3$ are defined as follows:

A:

(a)

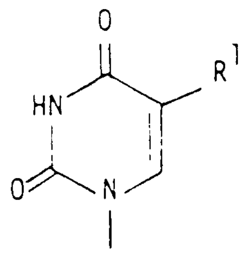

(b)

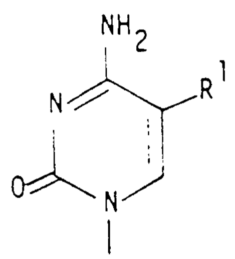

X:

(a) O
(b) S
(c) $CH_2$

$R^1$: H; alkyl containing 1-3 carbon atoms; $-CH=CH_2$;$-CH=CH-CH_3$;$-CH_2-CH=CH_2$ ;

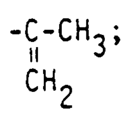

$-C \equiv CH$

$R^2$: H; or $R^2$ constitutes together with $R^3$ a carbon-carbon bond

$R^3$: H; F; Cl; Br; I; $N_3$; CN;$-C \equiv CH$; OH; $OCH_3$; $CH_2OH$; or $R^3$ constitutes together with $R^2$ a carbon-carbon bond,

and therapeutically acceptable salts thereof, inhibit the multiplication of human immunodeficiency virus (HIV). The compounds of the formula I are useful as therapeutic and/or prophylactic agents in the control and treatment of HIV virus infections in mammals and man.

In a more general aspect, the compounds of the formula I are useful as therapeutic and/or prophylactic

agents in the control and treatment of infections caused by retroviruses and hepatitis B virus in mammals and man.

All retroviruses, including HIV, require the enzyme reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermediate.

The compounds of the formula I inhibit the activity of reverse transcriptase of retroviruses including HIV as well as the activity of DNA polymerase of hepatitis B virus.

The present invention has several aspects:

1. the novel compounds included in the formula I,
2. pharmaceutical compositions comprising a compound of the formula I as active ingredient,
3. a compound of the formula I for use in therapy,
4. a compound of the formula I for use in the manufacture of a medicament for therapeutic and/or prophylactic treatment of infections caused by a retrovirus, including HIV, or by hepatitis B virus,
5. a method for the therapeutic and/or prophylactic treatment of infections in mammals and man caused by retrovirus including HIV or hepatitis B virus, by administering to a host in need of such treatment an efficient amount of a compound of the formula I.

It is a preferred aspect of the invention to combat HIV virus infections in man.

The expression "alkyl containing 1-3 carbon atoms" for the radical $R^1$ means $CH_3$, $C_2H_5$, $CH_2CH_2CH_3$, $CH(CH_3)_2$ and cyclopropyl.

When $R^3$ in formula I is F, Cl, Br, I, $N_3$, CN, C≡CH, OH, $OCH_3$ or $CH_2OH$ it may have either cis-configuration or trans-configuration relative to the hydroxymethyl function at position 4'.

Preferred compounds of the formula I are:

(a) A is

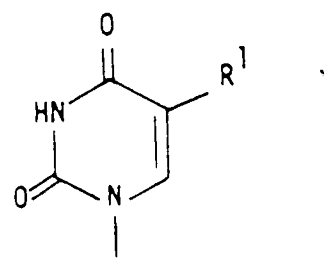

(b) A is

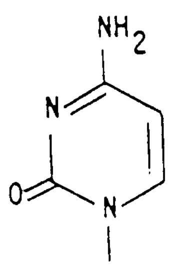

(c) $R^3$ at position 3' and the hydroxymethyl group at position 4' have the trans-configuration
(d) $R^1$ is $CH_3$ or $C_2H_5$
(e) X is O or $CH_2$
(f) X is O
(g) $R^2$ is H
(h) $R^2$ constitutes together with $R^3$ a carbon-carbon bond
(i) $R^3$ is H, F, $N_3$, OH, $OCH_3$, or $CH_2OH$ or constitutes together with $R^2$ a carbon-carbon bond
(j) $R^3$ is H, F, or $N_3$
(k) the combination of (a), (c), (d) and (e) above

(l) the combination of (a), (c), (d), (e), (g) and (i) above
(m) the combination of (a), (c), (d), (f), (g) and (j) above
(n) the combination (a), (c), (d), (e) and (h) above
(o) the combination (b), (c) , (d) and (e) above
(p) the combination (b), (c), (d), (e) , (g) and (i) above
(q) the combination (b), (c), (d), (f), (g) and (j) above
(r) the combination (b), (c), (d), (e) and (h) above

Examples of preferred compounds are:

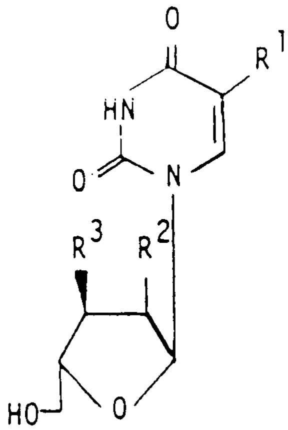

$R^1$ is $CH_3$; $R^2$ is H; $R^3$ is H
$R^1$ is $CH^3$; $R^2$ is H; $R^3$ is OH
$R^1$ is $CH_3$; $R^2$ is H; $R^3$ is $OCH_3$
$R^1$ is $CH_3$; $R^2$ is H; $R^3$ is $CH_2OH$
$R^1$ is $CH_3$; $R^2$ is H; $R^3$ is F
$R^1$ is $CH_3$; $R^2$ is H; $R^3$ is $N_3$
$R^1$ is $CH_3$; $R^2$ and $R^3$ constitute together a carbon-carbon bond
$R^1$ is $C_2H_5$; $R^2$ is H; $R^3$ is H
$R^1$ is $C_2H_5$; $R^2$ is H; $R^3$ is OH
$R^1$ is $C_2H_5$; $R^2$ is H; $R^3$ is $OCH_3$
$R^1$ is $C_2H_5$; $R^2$ is H; $R^3$ is $CH_2OH$
$R^1$ is $C_2H_5$; $R^2$ is H; $R^3$ is F
R1 is $C_2H_5$; $R^2$ is H; $R^3$ is $N_3$
$R^1$ is $C_2H_5$; $R^2$ and $R^3$ constitute together a carbon-carbon bond

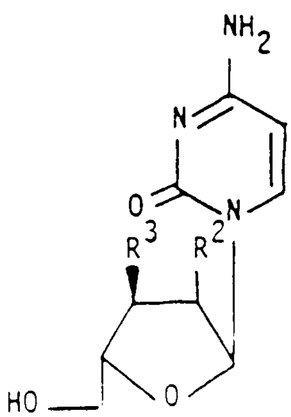

$R^2$ is H; $R^3$ is H
$R^2$ is H; $R^3$ is OH
$R^2$ is H; $R^3$ is $OCH_3$

$R^2$ is H; $R^3$ is $CH_2OH$
$R^2$ is H; $R^3$ is F
$R^2$ is H; $R^3$ is $N_3$
$R^2$ and $R^3$ constitute together a chemical bond
In all the examples of preferred compounds $R^3$ at position 3' and hydroxymethyl at position 4' have the trans-configuration.

In clinical practice the nucleosides of the formula I will normally be administered orally, by injection or by infusion in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule. The compound may also be used without carrier material. As examples of pharmaceutical preparations may be mentioned tablets, dragées, capsules, granulates, suspensions, elixirs, syrups, solutions etc. Usually the active substance will comprise between 0.05 and 20 % for preparations intended for injection and between 10 and 90 % for preparations intended for oral administration.

In the treatment of patients suffering from retrovirus, especially HIV, or hepatitis B virus infections, it will be preferred to administer the compounds by any suitable route including the oral, parenteral, rectal, nasal, topical and vaginal route. The parenteral route includes subcutaneous, intramuscular, intravenous and sublingual administration. The topical route includes buccal and sublingual administration. The dosage at which the active ingredients are administered may vary within a wide range and will depend on various factors such as the severity of the infection, the age of patient etc., and may have to be individually adjusted. As a possible range for the amount of the compounds of the invention or a physiologically acceptable salt thereof to be administered per day may be mentioned from about 10 mg to about 10 000 mg, preferentially 100 - 500 mg for intravenous administration and preferentially 100 - 3000 mg for oral administration.

Examples of pharmaceutically acceptable salts of the compounds of formula I include base salts, e.g. derived from an appropriate base, such as alkali metal (e.g. sodium), alkaline earth metal (eg. magnesium) salts, ammonium and $NX_4^+$ (wherein X is $C_{1-4}$ alkyl). Physiologically acceptable salts of a hydrogen atom or an amino group include salts of organic carboxylic acids such as acetic, lactic, gluconic, citric, tartaric, maleic, malic, panthothenic, isethionic, succinic, oxalic, lactobionic and succinic acids; organic sulfonic acids such as methanesulfonic, ethanesulfonic, benzenesulfonic, p-chlorobenzenesulphonic and p-toluenesulfonic acids and inorganic acids such as hydrochloric, hydroiodic, sulfuric, phosphoric and sulfamic acids. Physiologically acceptable salts of a compound of an hydroxy group include the anion of said compound in combination with a suitable cation such as $Na^+$, $NH_4^+$, and $NX_4^+$ (wherein X is a $C_{1-4}$ alkyl group).

Those compounds of the formula I which are novel are summarized as compounds of the formula I with the provisos that

1. when A, X, $R^2$ and $R^3$ are combined as follows:
A is

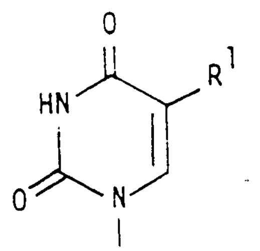

;

X is O;
$R^2$ is H;
$R^3$ is OH;
then $R^1$ is $-CH=CH_2$, $-CH=CH-CH_3$, $-CH_2-CH=CH_2$,

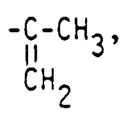

or cyclopropyl;

2. when A, X, $R^2$ and $R^3$ are combined as follows:
A is

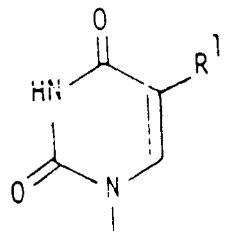

;

X is O;
$R^2$ is H;
$R^3$ is $N_3$;
then $R^1$ is H; alkyl containing 2-3 carbon atoms, $-CH=CH_2$; $-CH=CH-CH_3$; $-CH_2-CH=CH_2$;

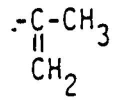
;

-C≡CH; or cyclopropyl;
3. when A, X, $R^2$ and $R^3$ are combined as follows:
A is

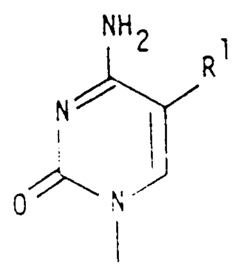

;

X is O;
$R^2$ is H;
$R^3$ is OH;
then $R^1$ is alkyl containing 1-3 carbon atoms, $-CH=CH_2$; $-CH=CH-CH_3$; $-CH_2-CH=CH_2$;

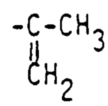
;

or cyclopropyl.

The administered compounds may also be used in therapy in conjunction with other medicaments such as 9-[(2-hydroxy-1-(hydroxymethyl)ethoxy)methyl]guanine, 9-(2-hydroxyethoxymethyl)guanine (acyclovir), 2-amino-9-(2-hydroxyethoxymethyl)purine, interferon, e.g., α-interferon, interleukin II, and phosphonoformate, or in conjunction with immune modulating therapy including bone marrow or lymphocyte transplants or medications such as levamisol or thymosin which would increase lymphocyte numbers and/or function as is appropriate.

Methods of preparation

The compounds of the invention may be prepared by one of the following general methods, constituting a further aspect of the invention.

A. Condensing a glycoside as comprised in formula I, where the hydroxyl groups may be optionally protected, to the N-1 position of a pyrimidine derivative, corresponding to radical A in formula I according to known methods described in the literature, followed by separation of the $\alpha$-anomer and removal of any protecting group(s). Such methods are described for example in "Basic Principles in Nucleic Acid Chemistry", Vol. 1 (Academic Press, 1974, Ed. P.O.P.Ts'o), in "Nucleoside Analogues, Chemistry, Biology and Medical Applications" (Pharma Press, 1979, Eds. R.T. Walker, E. De Clercq and F. Eckstein) and in Nucleic Acids Research Vol. 12, 1984, pages 6827 - 6837 (A.J. Hubbard, A.S. Jones and R.T. Walker). An example of such a method is given for the case of a uracil base analogue:

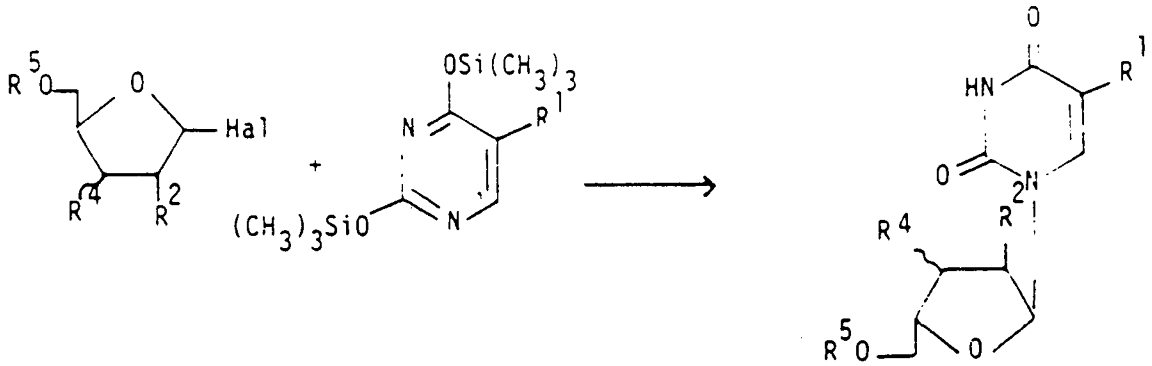

wherein $R^4$ is H, F, Cl, Br, I, $N_3$, CN, C=CH, $OR^5$, $OCH_3$ or $CH_2OR^5$, $R^5$ is a protecting group, of which a great variety is known, and examples of which are p-toluoyl, acetyl, trityl, benzyl. $R^1$ and $R^2$ are as defined above.

B. Anomerization of a $\beta$-anomer of the formula

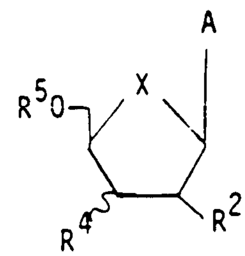

wherein A, X, and $R^2$ are as defined above, $R^4$ is H; F; Cl; Br; I; $N_3$; CN; $OR^5$; $OCH_3$; or $CH_2OR_5$; wherein $R^5$ is H or a hydroxy-protecting group to a mixture of $\alpha$- and $\beta$-anomers, whereafter the $\alpha$-anomer is separated and any protecting groups removed. The anomerization may be performed by known methods, e.g. with an optionally protected $\beta$-nucleoside, for example a silylated nucleoside, with a catalyst, such as for example trimethylsilyl trifluoromethanesulfonate

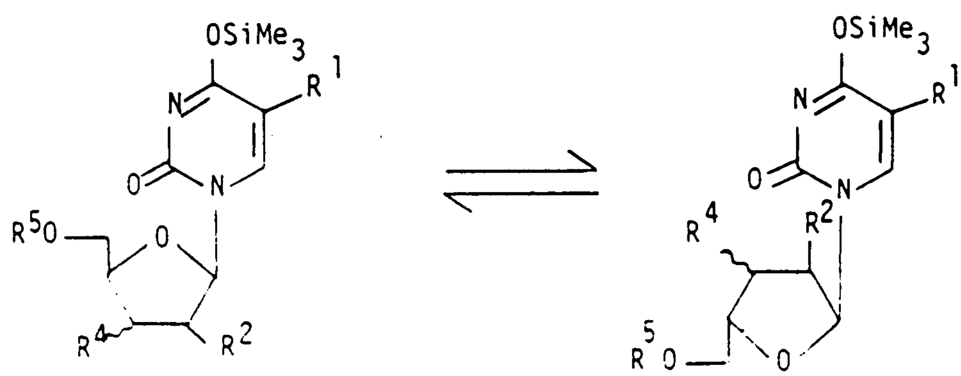

$R^1$, $R^2$, $R^4$ and $R^5$ are as defined above.

C. A transglycosylation reaction whereby the sugar moiety forming a bond, α- or β-, to one nucleoside base, is transferred to the desired pyrimidine base. The reaction is performed with a catalyst such as for example trimethylsilyl trifluoromethanesulfonate, and is followed by separation of the products and deprotection.

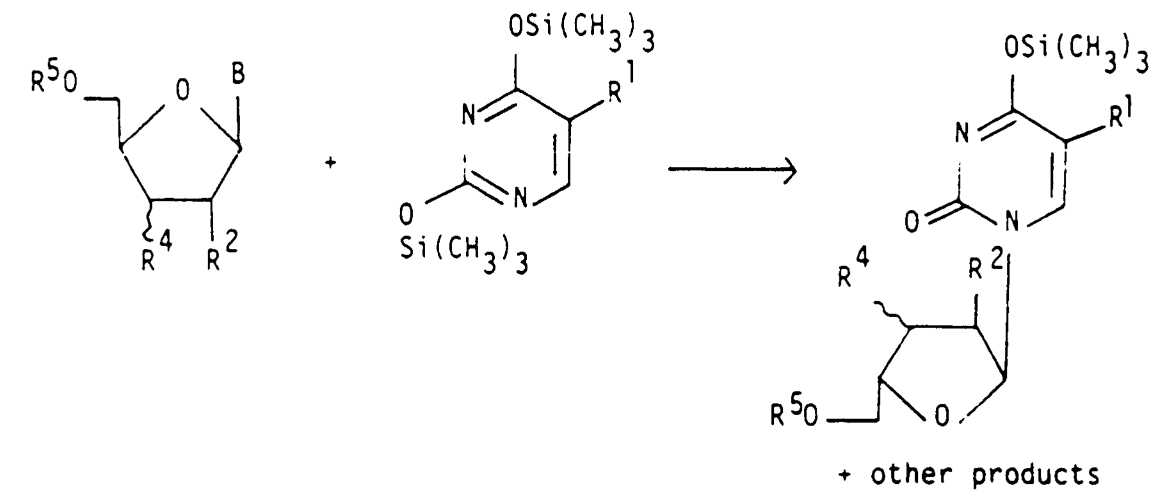


wherein $R^1$, $R^2$, $R^4$ and $R^5$ are as defined above. The radical B is a pyrimidine or purin base, the choice of which is not critical.

D. Introduction of the functional group $R^3$, or a precursor of $R^3$, into the nucleoside α-anomer by substitution of a suitable leaving group, $R^7$, followed by deprotection.

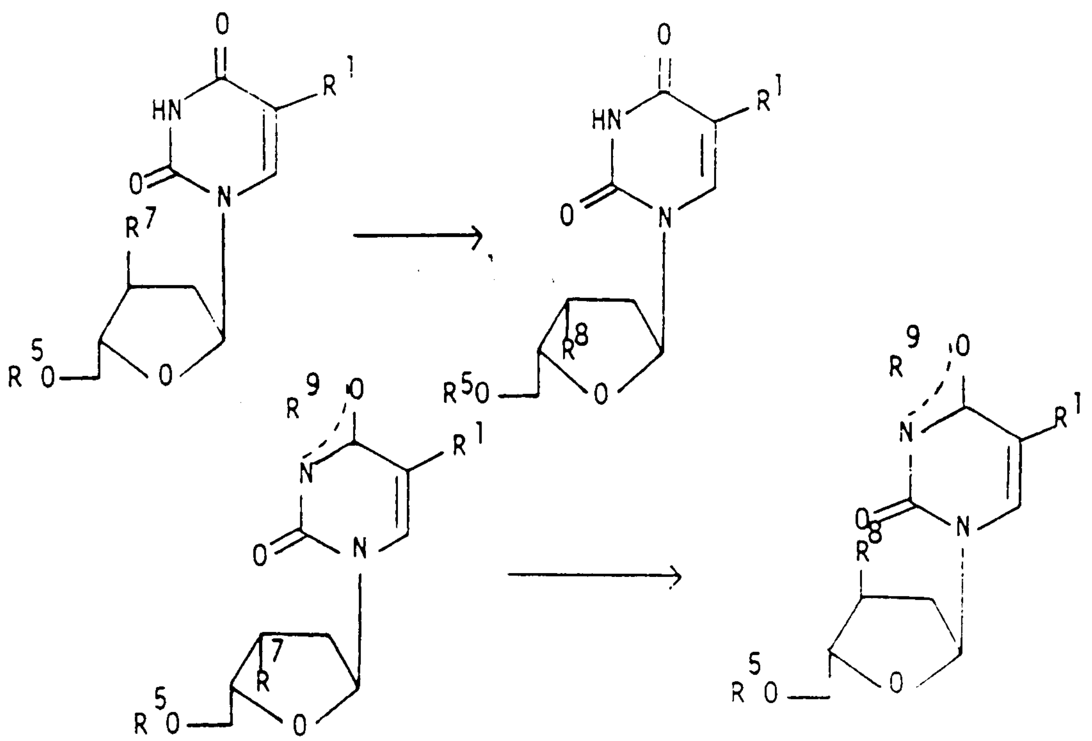

$R^1$ and $R^5$ are as defined above, $R^7$ is a good leaving group such as for example trifloromethanesulfonyloxy, $R^8$ is F, Cl, Br, I, $N_3$, CN, $OCH_3$ and synthos for the C≡CH, OH and $CH_2OH$ groups, such as for example $C≡C\text{-}Si(CH_3)_3$, $CH_3CO_2$ and

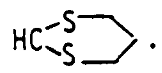

$R^9$ is a suitable protecting group.

An alternative way for introduction of the $R^8$ function is by reaction of the 2,3'-anhydro $\alpha$-anomer.

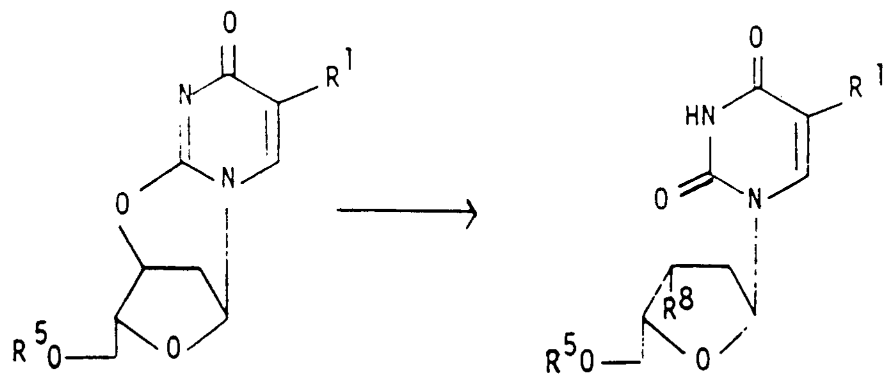

wherein $R^1$, $R^5$ and $R^8$ are as defined above.

The principles of methods A-D above are applicable to the synthesis of both uridine and cytidine analogues of formulas I and II, although the formulas illustrating the reactions only depict uridine analogues.

E. Converting the uracil moiety of the 5-substituted or unsubstituted $\alpha$-uridine compounds to a cytosine moiety of the corresponding $\alpha$-cytidine analogues. This is carried out by conventional methods, the principles of which have been described for example by W.L. Sung (J. Chem. Soc. Chem. Commun. 1981, p. 1089 and J. Organic Chemistry 1982, volume 47, pages 3623 - 3628) and by P. Herdewijn et al. (J. Medicinal Chemistry 1985, volume 28, pages 550 - 555).

The following examples will further illustrate the invention.

Preparation of intermediate products

A. Preparation of 3'F-3'-deoxy-5'-O-acetylthymidine (VSB423)

3'F-3'-deoxythymidine 45 mg (0.184 mmol) in acetic anhydride (2.0 mL) was heated with stirring in an oil bath at 80° for 7 hrs. The solution was evaporated in vacuo and the residual acetic anhydride and acetic acid were removed by several additions and reevaporations with benzenetoluene (1:1). The residue was used without further purifications.

Preparation of compounds of the invention

Example 1. Preparation of 1-(3-F-2,3-dideoxy-$\alpha$-D-ribofuranosyl)thymine (VSA 419) (Method B)

Thymine 23 mg (0.18 mmol) and 3'F-3'-deoxy-5'-O'-acetylthymidine was suspended in acetonitrile (1.2 mL) and N,O-Bis (trimethylsilyl)-acetamide (0.35 mL) was added. The mixture was stirred at room temperature for 1.5 hrs. Trimethylsilyl trifluoromethanesulfonate (0.05 mL) was added. After stirring at room temperature for 192 hrs, the mixture was poured under stirring into a 1:1 (v/v) mixture of 20 ml of 10 % aqueous $KHCO_3$-ethyl acetate. Two phases were separated and the water phase was extracted with ethyl acetate (3 x 10 mL). The combined ethyl acetate phase was filtered and evaporated in vacuo. The residue was dissolved in dichloromethane-ethyl acetate 1:1 and applied to a column of silica gel, and the column was eluated with dichloromethane-ethyl acetate 1:1 to give 28 mg (53 %) starting material (VSB 423) (Rf 0.37 on TLC silicagel $CH_2Cl_2$-EtoAc 1:1) and 18 mg (34 %) of 1-(3-F-2,3-dideoxy-5-O-acetyl-$\alpha$-D-ribofuranosyl)thymine (VSB 424) (Rf 0.29 on TLC silica gel $CH_2Cl_2$-EtoAc 1:1).

NMR ($CD_3OD$) $\delta$1.95 (s, 3H, $CH_3$-5), 2.12 (s, 3H, $CH_3CO$), 2.3-3.0 (m, 2H, H-2'a,b), 4.15 (d, 2H, J4' ,5' = 4.4 Hz, H-5'a,b), 4.81 (dt, 1H, J3'F,4' = 30.0 Hz, J4',5' = 4.6 Hz, H-4'), 5.23 (dd, 1H, J3'F,3' = 53.7 Hz, J2',3' = 5.0, H-3'), 6.36 (d, 1H, J1',2' = 7.57 Hz, H-1'), 7.27 (d, 1H, J H-6, $CH_3$ = 1.47, H-6)

$^{13}C(CD_3OD)$ δ12.80 ($CH_3$), 20.87 ($CH_3CO$), 39.40 (d, J = 20.8 Hz, C-2'), 63.37 (d, J = 12.2 Hz, C-5'), 84.65 (d, J = 24.4 Hz, C-4'), 86.50 (s, C-1') 93.82 (d, J = 178 Hz, C-3'), 111.12 (C-5), 135.07 (d, J = 6.1 Hz, C-6), 150.48 (C-2), 163.68 (C-4), 170.30 ($CH_3CO$).

The compound VSB 424 (16 mg) was dissolved in saturated methanolic ammonia (5 mL) and left at room temperature overnight. The solution was evaporated and the residue was treated with acetone-benzene (1:4) to give crystals of the desired compound, VSA 419 (9.4 mg, 69 %) UV λmax ($H_2O$) 269 nm. NMR (DMSO-d6) 'H δ1.79 (d, 3H, J $CH_3$, H-6 = 1.2 Hz $CH_3$), 2.16-2.90 (m, 2H, H-2'), 3.2-3.6 (m, 2H, H-5'), 4,61 (dt, 1H, J3'F,4' = 23.4 Hz, J4',5' = ~4 Hz, H-4'), 5.06 (t, 1H, J5',OH = 5.6 Hz, OH), 5.32 (dd, 1H, J3'F,3' = 54.2 Hz J2'3' = 4.9 Hz, H-3'), 6.18 (dd, 1H, J1'2' = 7.7 Hz and 2.1 Hz, H-1'), 7.39 (d, 1H, J $CH_3$, H-6 = 1.2 Hz, H-6)

$^{13}C$ (DMSO-d6) δ12.46 ($CH_3$),~39 (C-2'), 61.17 (d, J = 11.0 Hz, C-5'), 85.85 (C-1'), 87.15 (d, J = 20.8 Hz, C-4'), 94.75 (d, J = 173 Hz, C-3'), 109.15 (C-5), 135.63 (d, J = 6.1 Hz, C-6), 150.53 (C-2), 163.95 (C-4)

Example 2. Preparation of 1-(3-F-2,3-dideoxy-α-D-ribofuranosyl)-5-propyluracil (VSA 409) (Method C)

5-Propyluracil (56 mg) and 3'-F-3'deoxythymidine (47 mg) were suspended in acetonitrile (1.2 mL) and N,O-Bis (trimethylsilyl) acetamide (0.35 mL) was added. The mixture was stirred at room temperature for 1.5 hrs. Trimethylsilyl trifluoromethanesulfonate (0.05 mL) was added. After stirring at room temperature for 138 hrs, the mixture was evaporated in vacuo and added to $H_2O$ (0.5 mL), filtered and washed with $H_2O$ (0.5 mL). The combined water phase was applied to a $C_{18}$-column (HPLC) and eluted with methanol-water (35:65), at a rate of 7.0 ml/min. The β-anomer eluted after 12.9 min, and the desired α-anomer, VSA 409, after 18.0 min. Yield 9.3 mg (18 %), UV λ max ($H_2O$) 269 nm, MS $M^+$ 272 (10 %), 154 (100 %), 119 (76 %).

Example 3. Preparation of 1-(3-F-2,3-dideoxy-α-D-ribofuranosyl)-5-ethyluracil (VSA 411) (Method C)

5-Ethyluracil (51 mg) and 3'F-3'-deoxythymidine (48 mg) were suspended in acetonitrile (1.2 mL) and N,O-Bis (trimethylsilyl) acetamide (0.35 mL) was added. The mixture was stirred at room temperature for 1.5 hrs. Trimethylsilyl trifluoromethanesulfonate (0.05 mL) was added. After stirring at room temperature for 161 hrs, the mixture was evaporated in vacuo, and added to water (0.5 mL), filtered and washed with water (0.5 mL). The combined water phase was applied to a $C_{18}$-column (HPLC) and eluted with metnanol-water (1:3) at a rate of 8.0 ml/min. The β-anomer eluted after 12.3 min and the desired α-anomer, VSA 411, after 16.4 min. Yield 13.1 mg (26 %). UV λ max ($H_2O$) 267.5 nm. MS $M^+$ 258 (9 %), 140 (100 %), 119 (67 %).

Example 4. Preparation of 1-(2-deoxy-α-D-ribofuranosyl)-5-isopropenyluracil (VSA 175) (Method A)

5-Isopropenyluracil (4.3 g), hexamethyldisilazane (50 ml), chlorotrimethylsilane (1 ml) and ammoniumsulfate (catalytic amount) were heated at reflux for 2.5 hrs. Excess of solvent was evaporated in vacuo and the residual bis-silylated 5-isopropenyluracil (8.4 g) was dissolved in dichloroethane (50 ml) and added to 2-deoxy-3,5-di-O-p-toluoyl-D-erythro-pentosyl chloride (11.0 g) in dichloroetnane (150 ml) also containing molecular sieves (4 Å, 15 g). The suspension was stirred at room temperature overnight, after which it was filtered and the solvent was evaporated. The residue was redissolved in dichloromethane which was washed with saturated aq $NaHCO_3$ and $H_2O$, dried over $Na_2SO_4$ and concentrated to a volume of about 70 ml. A precipitate formed which was filtered off, dichloromethane was evaporated from the filtrate and the residue was subjected to chromatography on silica gel columns eluted with hexane/ethylacetate/dichloromethane (5/5/3), to give 1-(2-deoxy-3,5-di-O-p-toluoyl-α-D-ribofuranosyl)-5-isopropenyluracil (VSA 174), 2.64 g (Thin layer chromatography, silica gel, solvent system as above, Rf = 0.5).

Sodium metal (0.25 g) was dissolved in dry methanol (263 ml), compound VSA 174 (2.64 g) was added and the solution was stirred at room temperature overnight, after which water (35 ml) was added. The solution was neutralized with an ion exchanger (Dowex $H^+$ 50Wx2), filtered and the solvent was evaporated. The residue was washed with hexane and purified by chromatography on a column of silica RP18 eluted with 50 % aq methanol to give 1-(2-deoxy-α-D-ribofuranosyl)-5-isopropenyluracil. (TLC silica RP8, 50 % aq methanol, Rf = 0.5).

Biological tests

Test I. Effect of compounds of the formula I on HIV in H9 cells

Materials and methods: HIV infection of H9 cells

H9 cells, $10^5$ cells per well on a 24 well plate, suspended in 2 ml RPMI-medium containing 10 % fetal calf serum, 100 µg/ml penicillin, 10 µg/ml streptomycin sulfate and 2 µg/ml polybrene are exposed to HIV (HTLV-$III_B$) and different concentrations of the test compounds. The plates are incubated at 37°C in 5 % $CO_2$ for 6 - 7 days. The contents in each well is then homogenized with a pipette and transferred to a centrifuge tube. After centrifugation for 10 min at 1500 rpm the supernatent is removed and the cell pellet is analyzed by fixing in methanol on glass plates. Human HIV positive serum diluted 1:80 or 1:160 is added and incubated for 30 min at 37°. The plate is then washed with phosphate-buffered saline (PBS) containing $Ca^{2+}$ and $Mg^{2+}$. Sheep antihuman conjugate (FITC) is added and after a new incubation the plate is again washed with PBS. Contrast staining is done with Evans blue and after drying the frequency of HIV antigen containing cells is determined in a microscope. The test result is shown in Table 1.

Table 1

| Concentration (µM) for 50 % inhibition ($IC_{50}$) of human immuno deficiency virus multiplication in cell culture | |
|---|---|
| Compounds | $IC_{50}$(µM) |
| 1-(3-fluoro-2,3-dideoxy-α-D-ribofuranosyl)-5-ethyluracil (VSA 411) | 0.1 |
| 1-(3-fluoro-2,3-dideoxy-α-D-ribofuranosyl)-5-propyluracil (VSA 409) | 2.5 |
| 1-(2-deoxy-α-D-ribofuranosyl)-5-ethyluracil (VIP 289) | 10 |

It is seen in Table 1 that the tested compounds are active inhibitors of HIV virus multiplication.

Test II. Cellular toxicity

$H_9$ cells, $2x10^7$ cells per plate, are incubated in RPMI-1640 medium containing 10 % fetal calf serum, 70 mg/l penicillin, 100 mg/l streptomycin and 10 mM hepes, in absence or precence of test compounds. The number of cells per plate is determined after 48 hrs. Cells incubated in absence of test compound then underwent two cell division cycles.

F5000 cells, which are human embryo cells, $1x10^5$ cells per plate, are incubated in Eagle's minimal essential medium, supplemented with Earle's salts, non-essential amino acids, 10 % fetal calf serum, 10 mM hepes, 70 mg/l penicillin and 100 mg/l streptomycin, in absence or presence of test compounds. The number of cells per plate is determined after 48 hrs. Cells incubated in absence of test compounds underwent one cell division cycle. The results are given as $TC_{50}$, which is the concentration of a compound which gives 50 % inhibition of cell multiplication.

Table 2

| Cellular toxicity and H9 and F5000 cells | | |
|---|---|---|
| Compound | $TC_{50}$(µM) | |
| | H9 | F5000 |
| 1-(3-fluoro-2,3-dideoxy-α-D-ribofuranosyl)-5-ethyluracil (VSA 411) | 400 | 500 |
| 1-(3-fluoro-2,3-dideoxy-α-D-ribofuranosyl)-5-methyluracil (VSA 419) | | 250 |
| 1-(2-deoxy-α-D-ribofuranosyl)-5-ethyluracil (VIP 289) | | 1000 |

It is seen in Table 2 that the test compounds exhibit $TC_{50}$ values which vastly exceed the concentration $IC_{50}$ according to Table 1 to 50 % inhibition of HIV virus multiplication.

**Claims**

**1.** A compound of the formula

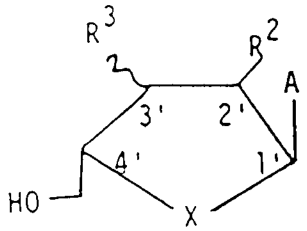

or

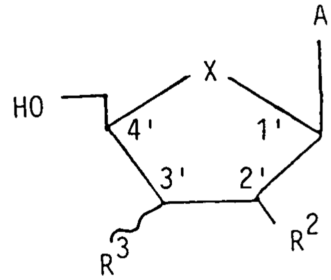

I

α-anomer β-anomer

wherein the radicals A, X, $R^2$ and $R^3$ are defined as follows

A:

(a)

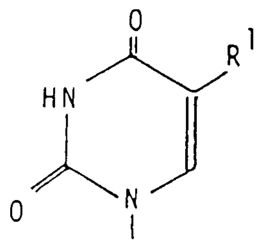

or

(b)

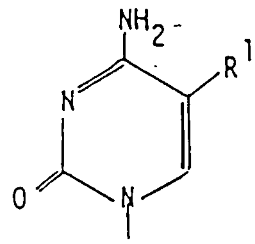

wherein $R^1$ is H, alkyl containing 1-3 carbon atoms, including cyclopropyl; $-CH=CH_2$; $-CH=CH-CH_3$; $-CH_2-CH=CH_2$;

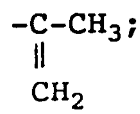

C = CH

X:

(a) S
(b) $CH_2$

$R^2$: H; or $R^2$ constitutes together with $R^3$ a carbon - carbon bond

$R^3$: H; F; Cl; Br; I; $N_3$; CN; C≡CH; OH; $OCH_3$; $CH_2OH$; whereby when $R^3$ in formula I is F; Cl; Br; I; $N_3$; CN; C≡CH; OH; $OCH_3$ or $CH_2OH$ it may have either cis-configuration or trans-configuration relative to the hydroxymethyl function at position 4', or $R^3$ constitutes together with $R^2$ a carbon - carbon bond, and therapeutically acceptable salts thereof.

**2.** A compound according to claim 1 wherein A is

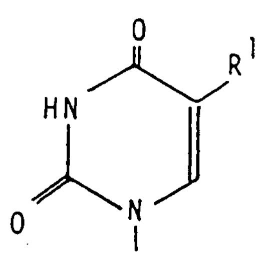

and $R^1$ is as defined in claim 1.

**3.** A compound according to claim 2 wherein $R^1$ is H, $CH_3$ or $C_2H_5$.

**4.** A compound according to claim 3 wherein $R^3$ is H; OH; $OCH_3$; $CH_2OH$; F; $N_3$ or $R^2$ and $R^3$ together constitute a carbon - carbon bond; and X is $CH_2$.

**5.** A compound according to claim 4 wherein $R^3$ at position 3' and hydroxymethyl at position 4' have the trans-configuration.

**6.** A compound according to claim 1 wherein A is

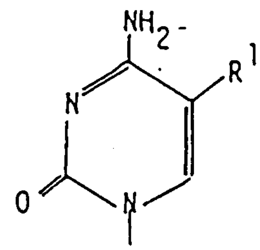

and $R^1$ is as defined in claim 1.

**7.** A compound according to claim 6 wherein $R^1$ is H; $CH_3$ or $C_2H_5$.

**8.** A compound according to claim 7 wherein $R^1$ is H.

**9.** A compound according to claim 8 wherein $R^3$ is H; OH; $OCH_3$; $CH_2OH$; F; $N_3$ or $R^2$ and $R^3$ together constitute a carbon - carbon bond; and X is $CH_2$.

**10.** A compound according to claim 9 wherein $R^3$ at position 3' and hydroxymethyl at position 4' have the trans-configuration.

**11.** A compound of the formula

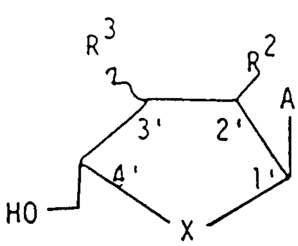

or

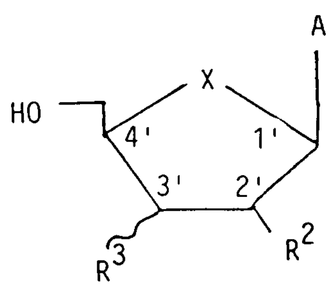

I

α-anomer β-anomer

wherein the radicals A, X, $R^2$ and $R^3$ are defined as in claim 1 and therapeutically acceptable salts thereof, for use in therapy.

**12.** A pharmaceutical composition comprising as active ingredient a compound of the formula

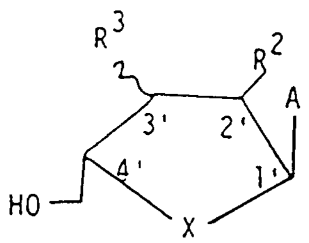

α-anomer

or

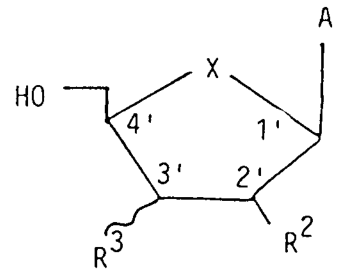

β-anomer

I

wherein the radicals A, $R^1$, $R^2$ and $R^3$ are defined as in claim 1 and therepeutically acceptable salts thereof.

**13.** A compound of the formula

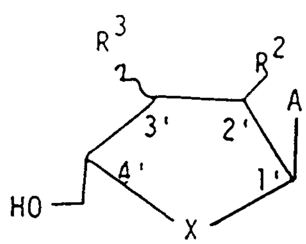

α-anomer

or

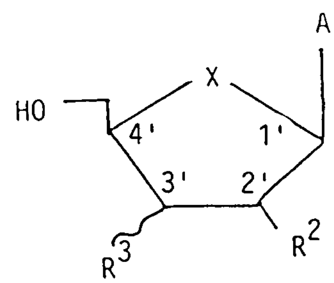

β-anomer

I

wherein the radicals A, X, $R^2$ and $R^3$ are defined as in claim 1 and therepeutically acceptable salts thereof, for use in the manufacture of a medicament for therapeutic and/or prophylactic treatment of infections caused by a retrovirus or by hepatitis B virus.

**14.** A compound according to claim 13 for use in the therapeutic treatment of infection in man caused by HIV virus.

**15.** A process for the preparation of a compound of the formula

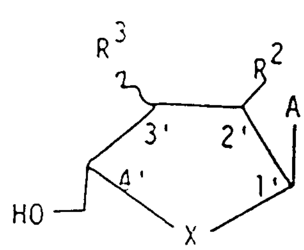

α-anomer

or

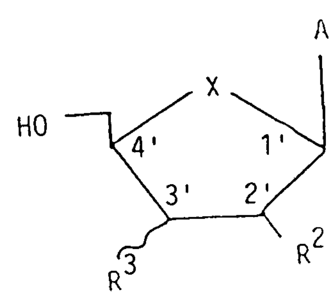

β-anomer

I

or a therapeutically acceptable salt thereof, wherein A, X, $R^2$ and $R^3$ are as defined in claim 1, by

A. Condensing a glycoside as comprised in formula I to the N-1 position of a pyrimidine derivative corresponding to radical A in Formula I, whereafter the α-anomer of compound I thus formed is separated and any protecting groups removed;

B. Anomerization of a β-anomer of the formula

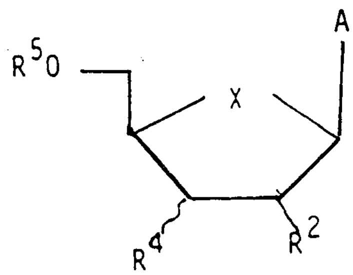

III

wherein A, X and $R^2$ are as defined above, and $R^4$ is H, F, Cl, Br, I, $N_3$, CN, $OR^5$, $OCH_3$ or $CH_2OR^5$ and $R^5$ is H or a hydroxy-protecting group, to a mixture of $\alpha$- and $\beta$-anomers, whereafter the $\alpha$-anomer is separated and any protecting groups removed;
C. Transglycosylation of a nucleoside of the formula

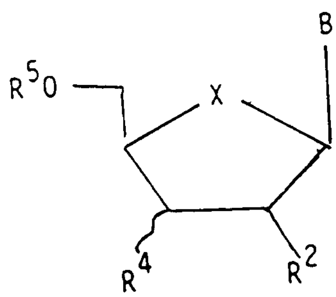

IV

wherein X, $R^2$, $R^4$ and $R^5$ are as defined above, and B is a pyrimidine or purine base, to the formation of a nucleoside containing the pyrimidine radical A as defined above, whereafter the $\alpha$-anomer is separated and any protecting groups removed;
D. Substitution of the radical $R^7$ in a compound of the formula

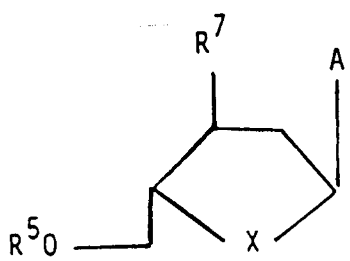

V

wherein A, X and $R^5$ are as defined above, and $R^7$ is a leaving group, with a radical $R^3$, whereafter any protecting groups are removed;
E. Conversion of the uracil moiety

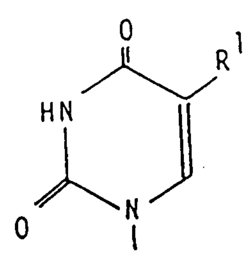

in a compound of the formula I to a cytosine moiety

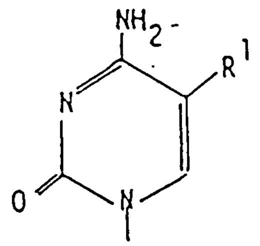

wherein $R^1$ is as defined above, whereafter the compound of the formula I thus obtained if desired is converted to a therapeutically acceptable salt.